# EUROPEAN PATENT APPLICATION

(11) **EP 3 492 495 A1**
(43) Date of publication of application: **05.06.2019**
(21) Application number: 18196113.7
(22) Date of filing: 12.05.2015
(51) Int. Cl.: C07K 16/22, A61M 5/31, A61K 39/395

(54) **PRE-FILLED PLASTIC SYRINGE CONTAINING A VEGF ANTAGONIST**

(30) Priority: 12.05.2014 EP 14167889
(62) Divisional of application: 15721021.2
(71) Applicant: Formycon AG, 82152 Martinsried/Planegg (DE)
(72) Inventor: FIEDLER, Bernd, 59427 Unna (DE)
(74) Representative: Maiwald Patent- und Rechtsanwaltsgesellschaft mbH

(57) **Abstract**

The present invention relates to a pre-filled syringe containing a VEGF antagonist and comprising a plastic barrel which is silicone-free, kits comprising this syringe and the use of the syringe for the administration of a VEGF antagonist in the treatment of ocular diseases.

## Description

### FIELD OF THE INVENTION

The present invention relates to a pre-filled syringe containing a VEGF antagonist and comprising a plastic barrel which is silicone-free, kits comprising this syringe and the use of the syringe for the administration of a VEGF antagonist in the treatment of ocular diseases.

### BACKGROUND OF THE INVENTION

Ocular diseases such as age-related macular degeneration and diabetic macular edema are caused by the uncontrolled growth of blood vessels in the eye. Hence, one option to treat these and similar diseases is to inhibit angiogenesis in the eye. Since VEGF is a key factor in the stimulation of angiogenesis, it is an attractive target for down-regulating angiogenesis.

Aflibercept, marketed under the name Eylea®, is a recombinant fusion protein consisting of the VEGF binding portion from the extracellular domains of human VEGF receptors 1 and 2 that are fused to the Fc portion of the human IgG1 immunoglobulin. It is approved for the treatment of wet macular degeneration. Ranibizumab, marketed under the name Lucentis®, is a Fab fragment of a humanized murine monoclonal antibody directed against VEGF and has been approved for the treatment of ocular diseases such as age-related macular degeneration and diabetic macular edema. In addition, the off-label use of the full-length antibody bevacizumab (Avastin®) which is also directed against VEGF for the treatment of ocular diseases is common. Ranibizumab and bevacizumab appear to have similar efficacy profiles in the treatment of neovascular age-related macular degeneration although rare adverse events seem to occur more often with bevacizumab (Johnson and Sharma (2013) Curr. Opin. Ophthalmol.: 24(3):205-12).

Both bevacizumab and ranibizumab are presented in glass vials from which they are usually drawn with a syringe shortly before injection into the eye. To use the whole content of the commercial vials of these antibodies, some companies repackage it in ready to use plastic syringes under sterile conditions, thereby allowing more than one syringe to be drawn from one glass vial. However, in the repackaged syringes silicone oil microdroplets and protein aggregates have been observed (Liu et al. (2011) Invest. Ophthalmol. Vis. Sci. 52(2): 1023-1034). Such silicone oil contaminants and protein aggregates may be responsible for the increase in intraocular pressure observed in patients treated with bevacizumab or ranibizumab (Kahook et al. (2009) Ophthalmic Surg. Lasers Imaging 40: 293-295; Good et al. (2011) Br. J. Ophthalmol. 95(8): 1111-1114).

AU 2012101677 A4 discloses pre-filled syringes containing a VEGF antagonist which syringes have a low silicone content. The whole disclosure of this document is focussed on the use of glass syringes and therefore teaches that a low amount of silicone has to be present within the syringe.

Further, recently a pre-filled ranibizumab syringe has been approved by the European Medicines Agency (EMA). The syringe barrel consists of borosilicate glass which was spray-coated with silicon oil-in-water emulsion and subsequently heat-fixed (so-called "baked silicone") (poster presentation by Clunas et al. at the 5th World Congress on Controversies in Ophthalmology, March 20-23, 2014; poster presentation of Michaud et al. at the ARVO Annual Meeting 2014).

Pre-filled syringes have many benefits compared to a vial and a separately provided syringe, such as improved convenience, affordability, accuracy, sterility, and safety. The use of pre-filled syringes results in greater dose precision, in a reduction of the potential for needle sticks injuries that can occur while drawing medication from vials, in pre-measured dosage reducing dosing errors due to the need to reconstite and/or draw medication into a syringe, and in less overfilling of the syringe helping to reduce costs by minimising drug waste.

However, glass syringes such as the approved ranibizumab pre-filled syringe are prone to breakage and have a relatively large weight compared to plastic syringes.

Further, they have to be treated with silicone to enable the correct movement of the stopper within the glass barrel and thereby effective and accurate drug delivery. It has been shown that silicone oil droplets occur in the vitreous cavity after intravitreal administration of VEGF antagonists and it was hypothesized that the silicone oil is derived from the needles and syringes used for the injections (Bakri and Ekdawi (2008) Retina 28: 996-1001).

Additionally, the glue which is necessary to attach a staked-in needle to a glass syringe can lead to impurities or increased protein oxidation (presentation of Adler at the 2011 PDA Europe The Universe of Pre-Filled Syringes and Injection Devices, Basel, 7-11 November 2011; presentation of Markovic at the PDA Single Use Systems Workshop, Bethesda, 22-23 June 2011).

Finally, during the manufacturing of glass pre-fillable syringes usually tungsten pins are used. It has been shown that soluble tungsten found in pre-filled syringes leads to protein aggregation and protein oxidation (Liu et al. (2010) PDA J. Pharm. Sci. Technol. 64(1): 11-19; Seidl et al. (2012) Pharm. Res. 29: 1454-1467).

Problems with glass pre-filled syringes have led to several product recalls in the past.

Several non-glass pre-filled syringes have been described. WO 2011/117878 A1 discloses a polycarbonate syringe, but it is not apparent whether the syringe barrel has been coated with silicone and whether the syringe is suitable for intraocular administration. WO 2009/099641 A2 discloses that in cyclic olefin polymer syringes without lubricant less visible particles form than in a glass syringe coated with silicone. However, it is not apparent whether this syringe can be used in ophthalmological applications.

Hence, there is still a need for non-glass syringes which can safely deliver the drug to the eye and which avoid the above disadvantages of using glass syringes, but in which the drug is stable for the storage period.

### SUMMARY OF THE INVENTION

The present inventors have surprisingly found that an anti-VEGF antibody solution is stable, i.e. the antibody is not significantly modified and does not aggregate significantly during storage when filled into a pre-filled syringe which comprises a silicone-free plastic syringe barrel, although it had been postulated that a plastic syringe is more permeable than a glass syringe for gases such as oxygen which may lead to protein modifications (see, e.g., Dierick and Yoshino (2015) OnDrugDelivery No. 55: 10-16). Hence, the syringe does not have to be packaged with an oxygen absorber. Further, the pre-filled syringe of the present invention does not contain a significant amount of particles. Finally, the forces required for injection of a solution from the pre-filled syringe of the present invention are comparable to the forces required for injection from a glass syringe.

The pre-filled syringe of the present invention therefore overcomes the disadvantages of glass syringes discussed above and may be used for administration of VEGF antagonists to the eye.

Accordingly, the present invention provides a pre-filled syringe containing a liquid formulation of a VEGF antagonist and comprising a syringe barrel, wherein the syringe barrel is made of plastic and is silicone-free.

In a preferred embodiment the VEGF antagonist is an anti-VEGF antibody or an antigen-binding fragment of such antibody or a soluble VEGF receptor fusion protein and more preferably the anti-VEGF antagonist is ranibizumab or aflibercept.

Preferably, the antagonist concentration is 1 to 100 mg/ml.

In one aspect of the invention the pre-filled syringe contains less than 50 particles per ml of the liquid formulation having a diameter of 10 µm or greater.

In another aspect of the invention the pre-filled syringe contains less than 5 particles per ml of the liquid formulation having a diameter of 25 µm or greater.

In still another aspect of the invention the pre-filled syringe has a glide force of less than or equal to 10N.

In a preferred embodiment the pre-filled syringe further comprises a silicone-free stopper.

Preferably, the syringe barrel is made of cycloolefin polymer or cycloolefin copolymer.

In a preferred embodiment the syringe barrel comprises an internal coating other than a silicone coating.

Also preferably, the pre-filled syringe comprises a staked needle.

The present invention also provides a kit comprising one or more pre-filled syringes according to the present invention. Preferably, the kit is a blister pack.

The pre-filled syringe may be used in administering a VEGF antagonist to a patient having an ocular disease, preferably having an ocular disease selected from the group consisting of age-related macular degeneration (AMD), visual impairment due to diabetic macular oedema (DME), visual impairment due to macular oedema secondary to retinal vein occlusion (branch RVO or central RVO), diabetic retinopathy in patients with diabetic macular edema or visual impairment due to choroidal neovascularisation (CNV) secondary to pathologic myopia.

Preferably, a volume of 30 to 100 µl of the liquid formulation is administered to the patient.

### BRIEF DESCRIPTION OF THE DRAWINGS

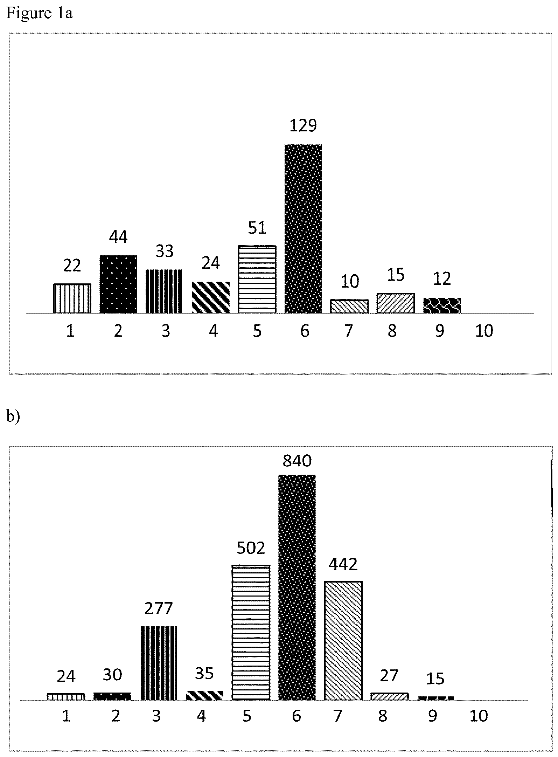

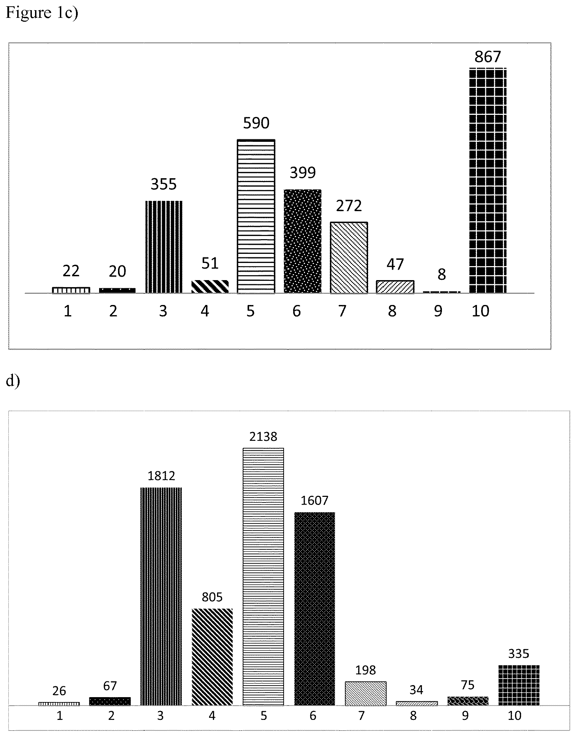

Figure 1: Number of particles with a diameter of 10 µm or greater after rotation from needle to stopper for 5 minutes (a), 2 weeks (b), 4 weeks (c) as well as after five freeze/thaw cycles (d) in the syringes listed in Table 1; syringe 10 was not measured in the analysis after 5 minutes and 2 weeks

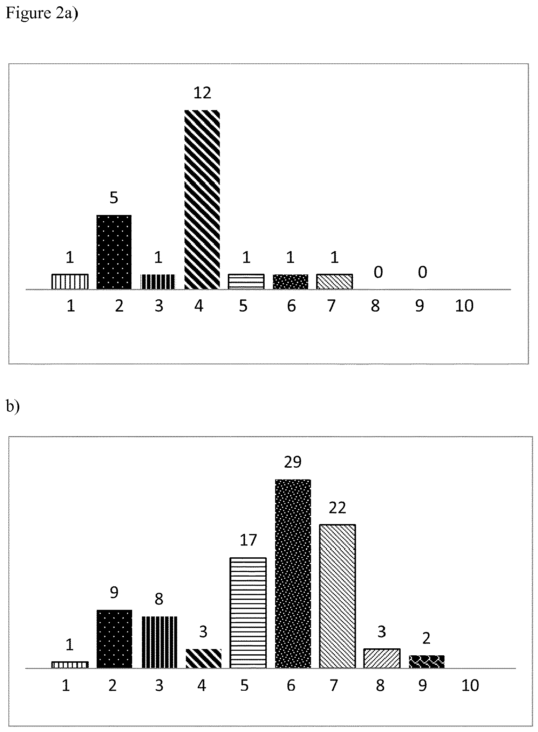

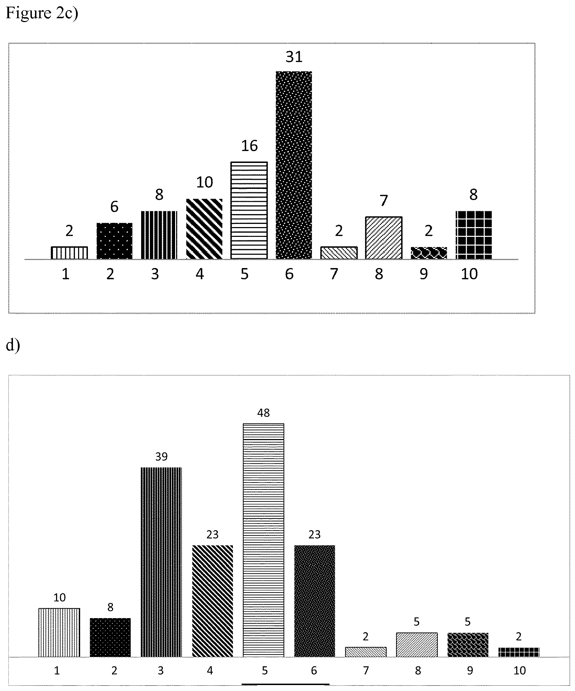

Figure 2: Number of particles with a diameter of 25 µm or greater after rotation from needle to stopper for 5 minutes (a), 2 weeks (b), 4 weeks (c) as well as after five freeze/thaw cycles (d) in the syringes listed in Table 1; syringe 10 was not measured in the analysis after 5 minutes and 2 weeks

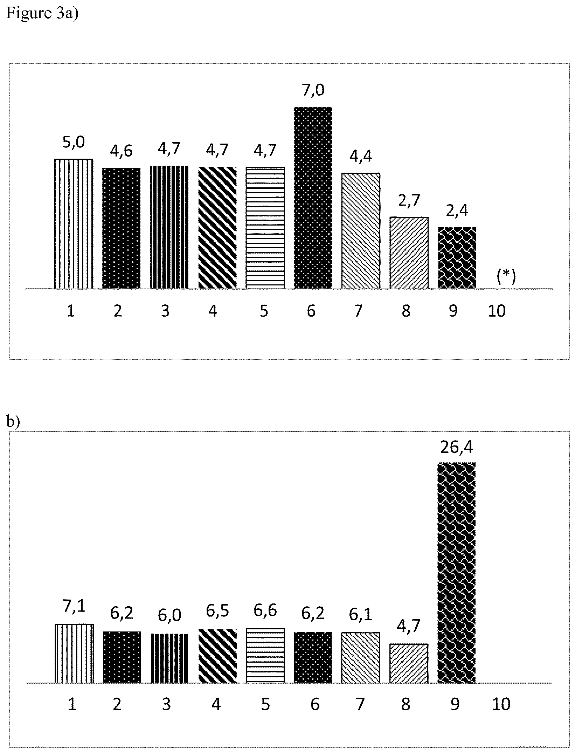

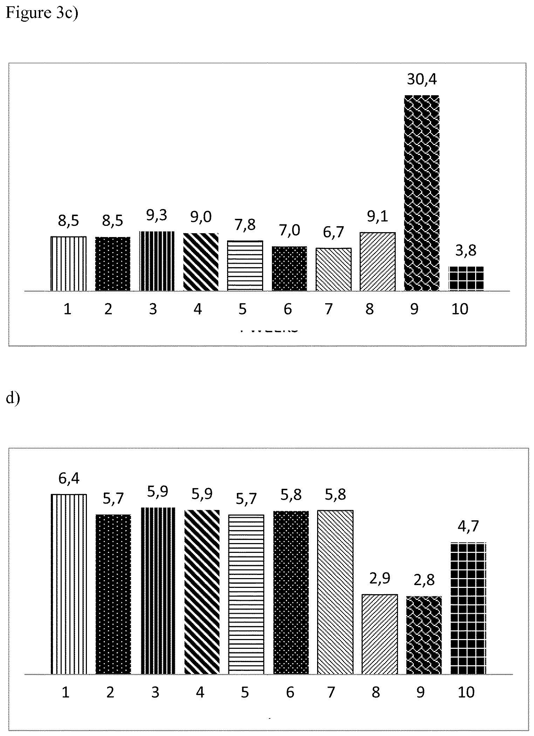

Figure 3: Percentage of hydrophilic species measured by RP-HPLC analysis of the content of the syringes listed in Table 1 after rotation from needle to stopper for 5 minutes (a), 2 weeks (b) and 4 weeks (c) as well as after five freeze/thaw cycles (d); syringe 10 was not measured in the analysis after 5 minutes and 2 weeks

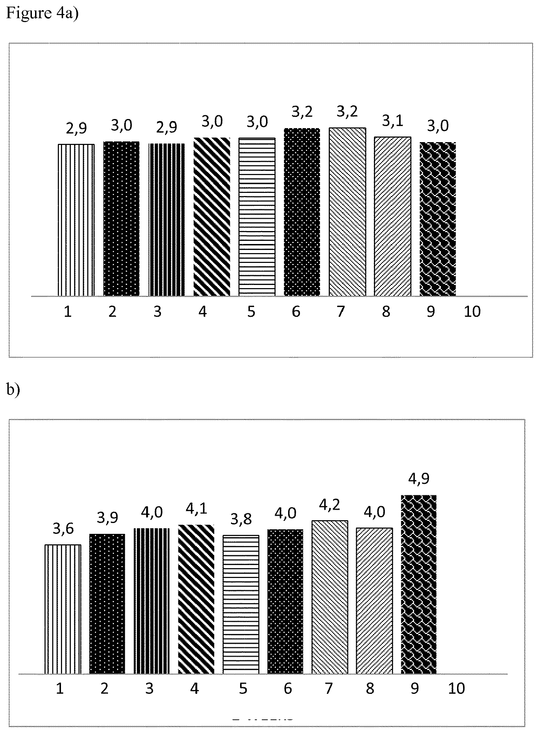

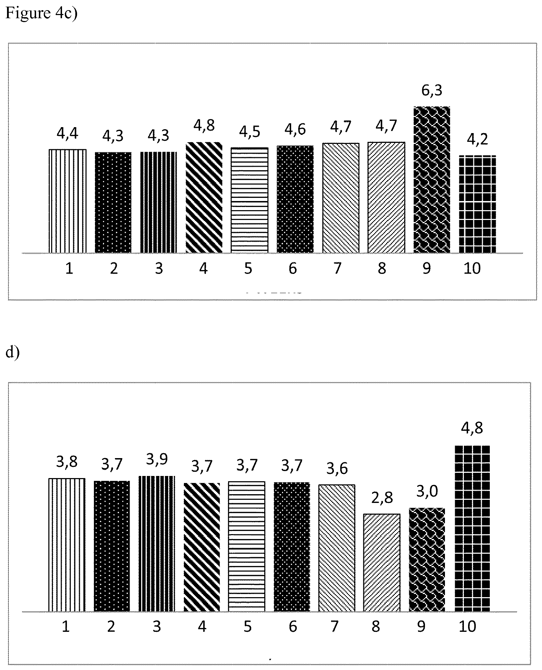

Figure 4: Percentage of hydrophobic species measured by RP-HPLC analysis of the content of the syringes listed in Table 1 after rotation from needle to stopper for 5 minutes (a), 2 weeks (b) and 4 weeks (c) as well as after five freeze/thaw cycles (d); syringe 10 was not measured in the analysis after 5 minutes and 2 weeks

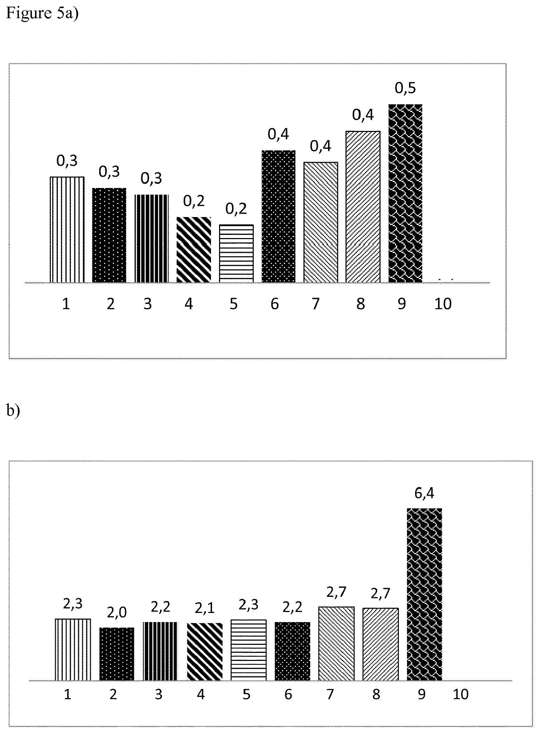

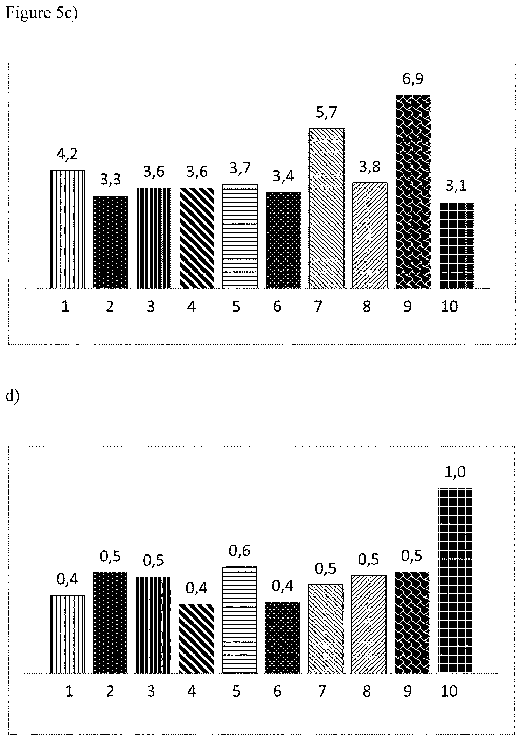

Figure 5: Percentage of acidic variants measured by cation exchange chromatography of the content of the syringes listed in Table 1 after rotation from needle to stopper for 5 minutes (a), 2 weeks (b) and 4 weeks (c) as well as after five freeze/thaw cycles (d); syringe 10 was not measured in the analysis after 5 minutes and 2 weeks

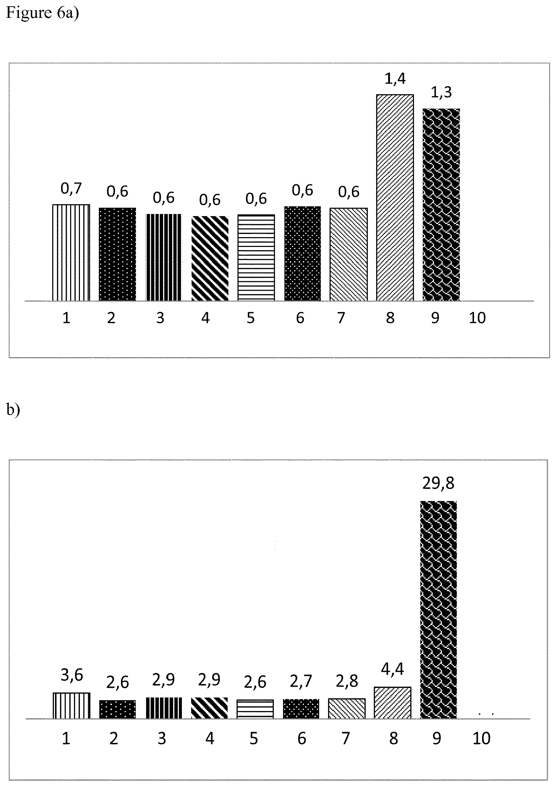

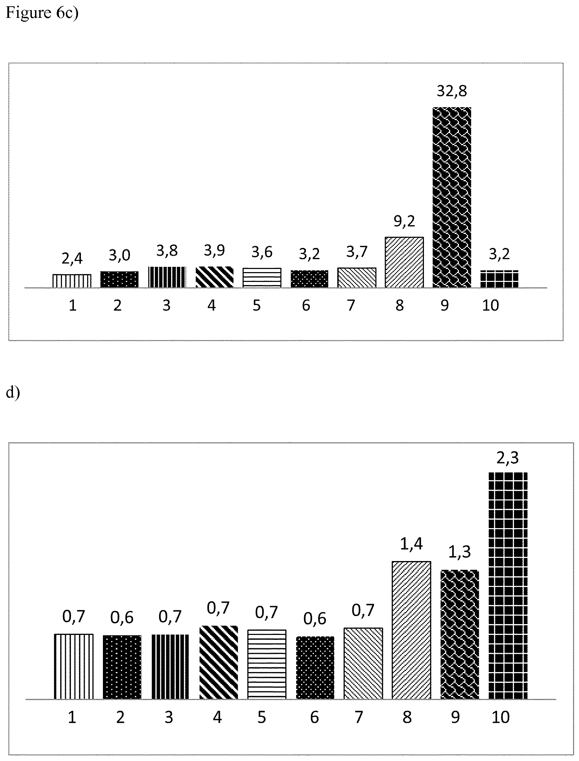

Figure 6: Percentage of basic variants measured by cation exchange chromatography of the content of the syringes listed in Table 1 after rotation from needle to stopper for 5 minutes (a), 2 weeks (b) and 4 weeks (c) as well as after five freeze/thaw cycles (d); syringe 10 was not measured in the analysis after 5 minutes and 2 weeks

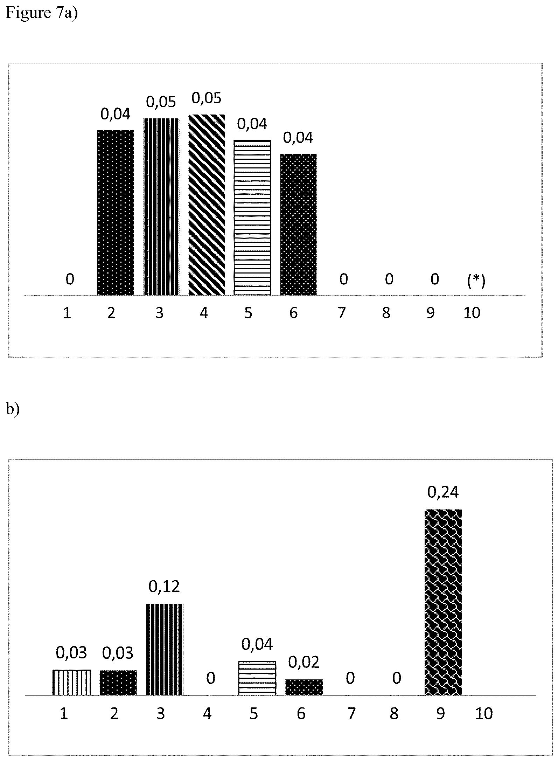

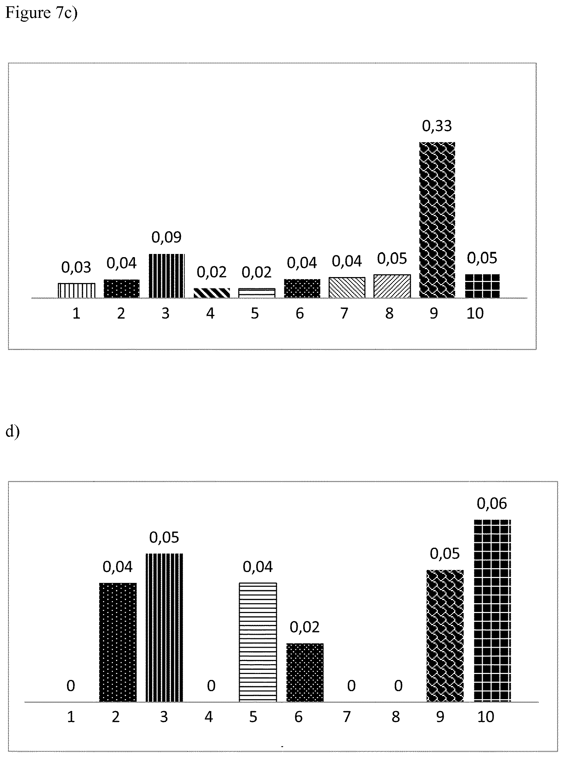

Figure 7: Analysis of protein aggregation by size exclusion chromatography after rotation from needle to stopper for 5 minutes (a), 2 weeks (b) and 4 weeks (c) as well as after five freeze/thaw cycles (d) of the content of the syringes listed in Table 1; syringe 10 was not measured in the analysis after 5 minutes and 2 weeks

### DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments, but the invention is not limited thereto, but only by the claims.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable".

Where an indefinite or definite article is used when referring to a singular noun, e.g. "a", "an" or "the", this includes a plural of that noun unless something else is specifically stated.

A "pre-filled syringe" is a syringe which is supplied by the manufacturer in a filled state, i.e. a measured dose of the drug to be administered is already present in the syringe when it is purchased and ready for administration. In particular, the pharmaceutical composition containing the drug does not have to be drawn from a vial containing the composition by using an empty syringe. The term pre-filled syringe within the meaning of the present invention does not refer to syringes the content of which has been drawn from a vial in a repackaging process.

The drug contained in the pre-filled syringe of the present invention, i.e. the VEGF antagonist, preferably an anti-VEGF antibody, is stable at a temperature of 2 to 8°C for at least six months, preferably for at least 9 months, more preferably for at least one year, particularly preferably for at least 18 months and most preferably for about two years. The drug contained in the pre-filled syringe of the present invention, i.e. the VEGF antagonist, preferably an anti-VEGF antibody or a VEGF receptor fusion protein and more preferably ranibizumab or aflibercept, is stable at room temperature, i.e. a temperature between 20°C and 25°C, for at least 24 hours, preferably for at least three days or one week, more preferably for at least two or three weeks, and most preferably for about 4 weeks. The drug contained in the pre-filled syringe of the present invention, i.e. the VEGF antagonist, preferably an anti-VEGF antibody or a VEGF receptor fusion protein and more preferably ranibizumab or aflibercept, is stable at a temperature of about 40°C, for at least 1 hour or 2 hours, preferably for at least four or six hours, more preferably for at least 10 or 12 hours, and most preferably for at least 18 or 24 hours.

The stability of the drug within the syringe can for example be determined by ion exchange chromatography by which modifications of the drug such as oxidized and deamidated species can be detected or by size exclusion chromatography by which aggregates of the drugs can be detected. A description of such an analysis is provided in the examples section.

The drug, i.e. the VEGF antagonist, preferably the anti-VEGF antibody, is considered stable, if the sum of all impurities comprising aggregates and chemically modified species is less than 2%, preferably less than 1.5%, more preferably less than 1.2% and most preferably less than 1% compared to the amount of non-modified, non-aggregated drug.

The drug contained in the pre-filled syringe of the present invention, i.e. the VEGF antagonist, preferably an anti-VEGF antibody or a VEGF receptor fusion protein and more preferably ranibizumab or aflibercept, retains its biological activity when stored at a temperature of 2 to 8°C for at least six months, preferably for at least 9 months, more preferably for at least one year, particularly preferably for at least 18 months and most preferably for about two years. The drug contained in the pre-filled syringe of the present invention, i.e. the VEGF antagonist, preferably an anti-VEGF antibody or a VEGF receptor fusion protein and more preferably ranibizumab or aflibercept, retains its biological activity when stored at room temperature, i.e. a temperature between 20°C and 25°C and 60% relative humidity for at least one day, preferably three days or one week, more preferably two weeks or three weeks and most preferably one month.. The drug contained in the pre-filled syringe of the present invention, i.e. the VEGF antagonist, preferably an anti-VEGF antibody or a VEGF receptor fusion protein and more preferably ranibizumab or aflibercept, retains its biological activity when stored at a temperature of about 40°C and 75% relative humidity for at least 1 hour or 2 hours, preferably for at least four or six hours, more preferably for at least 10 or 12 hours, and most preferably for at least 18 or 24 hours.

The biological activity of the VEGF antagonist, preferably an anti-VEGF antibody or a VEGF receptor fusion protein and more preferably ranibizumab or aflibercept can be determined by incubating different dilutions of the antagonist which was stored under the conditions described above with human umbilical vein endothelial cells (HUVEC) and VEGF and measuring the VEGF-induced proliferation of the cells in the presence of the antagonist, i.e. by the CellTiter-Blue® Cell Viability Assay available from Promega, in comparison to cells not incubated with the antagonist. Since the VEGF antagonist inhibits VEGF-induced signal transduction, the VEGF-induced proliferation will be reduced, if biologically active VEGF antagonist is present in the sample.

The VEGF antagonist, preferably the anti-VEGF antibody or VEGF receptor fusion protein and more preferably ranibizumab or aflibercept retains its biological activity after storage in the pre-filled syringe, such that the VEGF-induced proliferation is inhibited in HUVEC.

The components of a pre-filled syringe are known to a skilled person and basically comprise a syringe barrel and a plunger.

The syringe barrel contains a defined volume of the liquid composition which can be expelled from the barrel through an outlet positioned on one end of the barrel when the plunger is pushed into and moves along the barrel. The syringe barrel typically has a substantially cylindrical shape. The outlet may comprise a projection from the outlet end through which extends a channel having a smaller diameter than the rest of the syringe barrel. The outlet may be adapted, for example by a luer lock type connection, for connection with a needle or other accessory such as a sealing device which is able to seal the barrel and can be removed to allow a needle to be attached to the syringe. This sealing can be achieved by the use of known sealing devices such as the OVS™ system of Vetter Pharma International GmbH.

In the pre-filled syringe of the present invention the syringe outlet is firmly connected with a needle so that the pre-filled syringe is supplied with a staked needle and does not need to be assembled prior to use. In this case, the risk of injuries with the needle during assembly of the syringe before injection is reduced. The staked needle can be attached to the pre-filled plastic syringe of the present invention without using an adhesive, since it can be moulded into the syringe. In contrast, an adhesive is required to attach the needle to a glass syringe and can lead to impurities or increased protein oxidation (presentation of Adler at the 2011 PDA Europe The Universe of Pre-Filled Syringes and Injection Devices, Basel, 7-11 November 2011; presentation of Markovic at the PDA Single Use Systems Workshop, Bethesda, 22-23 June 2011).

For intravitreal administration the needle size is typically 30 gauge, although 31-, 32, 33- and 34-gauge needles may also be used. The pre-filled syringe may be equipped with a passive needle safety guard to further avoid the danger of needle sticks after injection.

The pre-filled syringe of the present invention comprises a syringe barrel which is made from plastic material. Preferably, the plastic material is selected from cycloolefin polymer and cycloolefin copolymer.

Cycloolefin copolymers may be produced by chain copolymerization of cyclic monomers such as 8,9,10-trinornorn-2-ene or 1,2,3,4,4a,5,8,8a-octahydro-1,4:5,8-dimethanonaphthalene with ethane. Suitable copolymers are those of the Topas™ type which are available in a variety of grades.

Cycloolefin polymers may for example be produced by ring-opening metathesis polymerization of various cyclic monomers followed by hydrogenation. Suitable commercially available containers made of cycloolefin polymer material include containers manufactured from CZ™ resin, Zeonor™ and Zeonex™.

According to the present invention the syringe barrel is silicone-free which means that the inner surface of the syringe barrel has not been treated with silicone oil. Hence, no silicone oil can be detected within the pre-filled syringe of the present invention.

The presence and thickness of silicone layers can be determined by known methods such as the rap.ID Layer Explorer® application which can also be used to measure the amount of silicone oil within the syringe barrel. The amount of silicone oil within the syringe barrel can also be measured by differential weighing methods and quantitation by infrared spectroscopy of the oil diluted in a suitable solvent.

Preferably, the pre-filled syringe is uncoated, i.e. the cycloolefin polymer or copolymer material is in direct contact with the liquid composition contained therein and the syringe barrel does not contain any material other than the plastic material of which the syringe is made.

Alternatively, the pre-filled syringe may comprise an internal coating other than a silicone coating. The term "internal coating" is intended to mean a coating on the inner side of the syringe barrel which is in contact with the drug solution. Examples of such an internal coating include a fluorocarbon film made from a modified ethylene-tetrafluoroethylene copolymer (also called Flurotec® film, available from West Pharmaceutical Services) and a perfluoropolyether film crosslinked by an Atmospheric Plasma Immobilization™ process (also called TriboGlide®, available from TriboFilm Research and described in WO 2005/094214 A2). Another example of an internal coating is a silicon-oxide barrier coating applied to the plastic surface. To the silicone oxide layer further coating layers may be applied, resulting in a multilayer coating. Such plastic syringes coated with a silicon oxide barrier coating and optionally further coating layers are available from SiO₂ and described in ONdrug Delivery Magazine issue 45, October 2013 and issue 47, February 2014 as well as in WO 2014/059012 A1.

The pre-filled syringe may also comprise a layer within the plastic body of the syringe so that the syringe comprises from the outside to the interior three layers: an external plastic layer which is in contact with the environment, an intermediate layer made of a material other than the external and internal plastic layers and an internal plastic layer which is made of the same or another plastic material than the external layer and which is in contact with the drug solution. The intermediate layer may comprise an oxygen-absorbing resin so that the syringe may comprise from the outside to the interior the following three layers: cycloolefin copolymer - oxygen-absorbing resin - cycloolefin copolymer. Such syringes are marketed under the name Oxy-Capt™ by Mitsubishi Gas Chemical Company and are for example described in WO 2014/136914 A1.

Alternatively, the syringe may also comprise a coating on the outer surface of the syringe which is in contact with the environment such as an oxygen barrier coating. The syringe barrel is also tungsten-free, i.e. it does not contain any traces of tungsten, since it is not necessary to use tungsten in the syringe manufacturing process. Hence, there is no risk of tungsten-induced protein aggregation.

In one embodiment the syringe barrel comprises a mark such as a line printed on the syringe barrel which line allows the person injecting the liquid composition to align a pre-determined part of the stopper (such as the tip of the front surface) or plunger with the mark. Thereby, any excess liquid composition and potential air bubbles are removed from the syringe barrel, allowing the safe administration of an exact predetermined dosage to the patient.

The plunger is pulled and pushed along inside the syringe barrel, allowing the syringe to expel the liquid formulation through the outlet.

At the end of the plunger which is in contact with the liquid formulation a stopper is located. The stopper portion is typically made of an elastomeric material such as natural or synthetic rubber, which engages an inner surface of the syringe barrel to create a seal that facilitates ejecting the liquid formulation from the syringe when pressure is applied to the plunger.

The stopper may be coated with a fluoropolymer film such as FluroTec® barrier film or a polytetrafluoroethylene-like film such as an Omniflex stopper. This type of coating serves as an effective barrier between the drug and the elastomer, reducing the potential for extractables or leachables which are inherent to all materials. In addition, the coating reduces the occurrence of the reverse process, where the drug product can adsorb or absorb into the plunger. The stopper is preferably silicone-free, i.e. at least the surface of the stopper which comes into contact with the drug solution and more preferably the complete stopper has not been coated with silicone oil.

The syringe has a nominal maximum fill volume, i.e. a volume which can be maximally taken up by the syringe, of 0.3 ml to 1.5ml, preferably of 0.5 ml to 1.0 ml, most preferably 0.5 ml or 1.0 ml. The volume of the liquid composition filled into the syringe is about 0.05 ml to about 1ml, preferably about 0.1 ml to about 0.5 ml, more preferably 0.14 ml to 0.3 ml and most preferably 0.15 ml to 0.2 ml.

The skilled person knows that the syringe is usually filled with a volume which is larger than the volume actually administered to the patient to take into account any dead space within the syringe and the needle and the loss due to the preparation of the syringe for injection. Hence, the volume which is actually administered to the patient is between 0.01 ml and 1 ml, preferably between 0.02 and 0.5 ml, more preferably between 0.025 and 0.5 ml, even more preferably between 0.03 ml and 0.05 ml and most preferably the volume which is actually administered to the patient is 0.05 ml.

Ranibizumab is typically administered in a volume of 0.05 ml with a ranibizumab concentration of 6 or 10 mg/ml or in a volume of 0.03 ml or 0.05 ml with a ranibizumab concentration of 10 mg/ml, yielding a delivered amount of 0.3 or 0.5 mg. For aflibercept the administered volume is typically 0.05 ml with an aflibercept concentration of 40 mg/ml, yielding a delivered amount of 2 mg. As discussed above, bevacizumab is used off-label for the treatment of ocular diseases. In this case, the administered volume of bevacizumab is 0.05 ml with a bevacizumab concentration of 25 mg/ml, yielding a delivered amount of 1.25 mg.

Hence, in one embodiment the syringe is filled with a volume of the liquid composition of 0.15 ml to 0.2 ml and 0.03 ml to 0.05 ml of the liquid composition are administered to the patient.

The term "VEGF antagonist" refers to a molecule which specifically interacts with VEGF and inhibits one or more of its biological activities, e.g. its mitogenic, angiogenic and/or vascular permeability activity. It is intended to include both anti-VEGF antibodies and antigen-binding fragments thereof and non-antibody VEGF antagonists.

Non-antibody VEGF antagonists include aflibercept, pegaptanib and antibody mimetics. Preferably, the non-antibody VEGF antagonist is aflibercept. Aflibercept which is presently marketed under the name Eylea® and which is also known as VEGF-trap is a recombinant human soluble VEGF receptor fusion protein in which portions of human VEGF receptors 1 and 2 extracellular domains are fused to the Fc portion of human IgG1 (Holash et al. (2002) Proc. Natl. Acad. Sci. USA 99(17): 11393-11398; WO 00/75319 A1). It has received a marketing authorization for the treatment of wet age-related macular degeneration, visual impairment due to diabetic macular oedema (DME) and diabetic retinopathy in patients with diabetic macular edema. The present commercial aflibercept formulation contains sodium phosphate, sodium chloride, polysorbate 20, sucrose and water for injection and is supplied in a concentration of 40 mg/ml.

Pegaptanib which is presently marketed under the name Macugen® is a pegylated anti-vascular endothelial growth factor (VEGF) aptamer (Bell et al. (1999) In Vitro Cell Dev Biol Anim. 35(9): 533-42). Antibody mimetics which are VEGF antagonists include binding proteins comprising an ankyrin repeat domain that binds VEGF and inhibits its binding to the receptor, such as DARPin® MP0112 (see also WO 2010/060748 and WO 2011/135067).

The term "anti-VEGF antibody" refers to an antibody or antibody fragment such as a Fab or a scFV fragment that specifically binds to VEGF and inhibits one or more of its biological activities, e.g. its mitogenic, angiogenic and/or vascular permeability activity. Anti-VEGF antibodies act, e.g., by interfering with the binding of VEGF to a cellular receptor, by interfering with vascular endothelial cell activation after VEGF binding to a cellular receptor, or by killing cells activated by VEGF. Anti-VEGF antibodies include, e.g., antibodies A4.6.1, bevacizumab, ranibizumab, G6, B20, 2C3, and others as described in, for example, WO 98/45331 , US 2003/0190317, US 6,582,959, US 6,703,020, WO 98/45332, WO 96/30046, WO 94/10202, WO 2005/044853, EP 0 666 868 B1, WO 2009/155724 and Popkov et al. (2004) J. Immunol. Meth. 288: 149-64. Preferably, the anti-VEGF antibody or antigen-binding fragment thereof present in the pharmaceutical composition of the present invention is ranibizumab or bevacizumab. Most preferably, it is ranibizumab or an antigen-binding fragment thereof.

"Ranibizumab" is a humanised monoclonal Fab fragment directed against VEGF-A having the light and heavy chain variable domain sequences of Y0317 as described in SEQ ID Nos. 115 and 116 of WO 98/45331 and Chen et al. (1999) J. Mol. Biol. 293: 865-81. The CAS number of ranibizumab is 347396-82-1. Ranibizumab inhibits endothelial cell proliferation and neovascularisation and has been approved for the treatment of neovascular (wet) age-related macular degeneration (AMD), the treatment of visual impairment due to diabetic macular oedema (DME), the treatment of visual impairment due to macular oedema secondary to retinal vein occlusion (branch RVO or central RVO), or treatment of visual impairment due to choroidal neovascularisation (CNV) secondary to pathologic myopia. Ranibizumab is related to bevacizumab and derived from the same parent mouse antibody as bevacizumab but it is much smaller than the parent molecule and has been affinity matured to provide stronger binding to VEGF-A. Ranibizumab is produced recombinantly in *Escherichia coli*, e.g. as described in WO 98/45331 A2. The present commercial ranibizumab formulation contains α,α-trehalose dihydrate, histidine hydrochloride monohydrate, histidine, polysorbate 20 and water for injection and is supplied in a concentration of 10 mg/ml.

"Bevacizumab" is a full-length, humanized murine monoclonal antibody that recognizes all isoforms of VEGF and which is the parent antibody of ranibizumab. The CAS number of bevacizumab is 216974-75-3. Bevacizumab inhibits angiogenesis and is presently approved for the treatment of different cancer types. However, it is also used off-label in ophthalmological diseases such as age-related macular degeneration. The present commercial bevacizumab formulation contains α,α-trehalose dihydrate, sodium phosphate, polysorbate 20 and water for injection and is supplied as a concentrate with a concentration of 25 mg/ml.

The antibody concentration within the pre-filled syringes of the present invention is typically 1-100 mg/ml, preferably 2-75 mg/ml, more preferably 3-50 mg/ml, even more preferably 5 to 30 mg/ml and most preferably 6 or 10 mg/ml. If ranibizumab is contained within the pre-filled syringe of the present invention the ranibizumab concentration is 10 mg/ml.

The liquid composition within the pre-filled syringe of the present invention has a low particle content. In particular, it comprises less than 50 particles having a size of more than 10 µm after the syringe has been rotated at 40°C for five minutes, two weeks or four weeks or after three freeze-thaw cycles from +5°C to -20°C with 1°C per minute or after storage of the syringe at 5°C or 25°C and 60% relative humidity for three months. Alternatively or additionally, it comprises less than 5 particles having a size of more than 25 µm after the syringe has been rotated at 40°C for five minutes, two weeks or four weeks or after three freeze-thaw cycles from +5°C to - 20°C with 1°C per minute or after storage of the syringe at 5°C or 25°C/60% relative humidity for three months. Hence, the pre-filled syringe meets the requirements of United States Pharmacopoiea <789> for ophthalmic solutions with respect to these particle sizes.

The pre-filled syringe of the present invention further has excellent gliding behaviour. In particular, the break loose force, i.e. the force required to initiate the movement of the plunger, is less than 10N or 9N, preferably less than 8N or 7N, more preferably less than 6N and most preferably less than 5N. The break loose force does not change significantly, i.e. by more than 10%, when the syringe is stored for an extended period such as eight weeks. In contrast, in a syringe containing silicone the break loose force increases upon storage by at least twofold.

Further, the gliding force, i.e. the force required to sustain the movement of the plunger along the syringe barrel to expel the liquid composition, is less than 10N, preferably less than 9N, more preferably less than 8N and most preferably less than 7N. In a particularly preferred embodiment there is no significant difference between the break loose force and the gliding force.

The present invention also provides a kit comprising one or more of the pre-filled syringes of the present invention. Preferably, the kit comprises a blister pack. A "blister pack" has a cavity or pocket which is usually made from thermo formed plastic and a backing of paperboard or a lidding seal of aluminium foil or plastic. The blister pack may be aseptically sterile before the sterile syringe is packaged into it under aseptic conditions. Hence, no sterilization after packaging is required. The kit may further comprise a needle, if the pre-filled syringe does not comprise a staked-in needle. The kit may further comprise instructions for use.

Preferably, the kit does not comprise an oxygen absorber which is typically used to reduce the level of oxygen within a package such as a blister pack. Oxygen absorbers usually contain a substance such as ferrous carbonate or ascorbate which substance reacts with any oxygen within a package with a high affinity, thereby reducing the oxygen content of the package.

An "intraocular neovascular disease" is a disease characterized by ocular neovascularisation. Examples of intraocular neovascular diseases include, e.g., proliferative retinopathies, choroidal neovascularisation (CNV), age-related macular degeneration (AMD), diabetic and other ischemia-related retinopathies, diabetic macular oedema, diabetic retinopathy in patients with diabetic macular edema, pathological myopia, von Hippel-Lindau disease, histoplasmosis of the eye, Central Retinal Vein Occlusion (CRVO), Branch Retinal Vein Occlusion (BRVO), corneal neovascularisation, and retinal neovascularisation. The term "age-related macular degeneration" refers to a medical condition which usually affects older adults and results in a loss of vision in the centre of the visual field (the macula) because of damage to the retina.

Preferably, the pre-filled syringe is for use in the intravitreal injection of a VEGF antagonist as defined herein.

The term "intravitreal injection" refers to the administration of a pharmaceutical composition in which the substance is injected directly into the eye. More specifically, the substance is injected into the vitreous humour (also called vitreous body or simply vitreous) which is the clear gel that fills the space between the lens and the retina of the eyeball of humans and other vertebrates.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

The detailed description is merely exemplary in nature and is not intended to limit application and uses. The following examples further illustrate the present invention without, however, limiting the scope of the invention thereto. Various changes and modifications can be made by those skilled in the art on the basis of the description of the invention, and such changes and modifications are also included in the present invention.

### EXAMPLES

### 1. Determination of particles of different sizes in different plastic and glass syringes containing ranibizumab and subjected to different conditions

400 µl of a solution of the anti-VEGF antibody ranibizumab containing 1 mg/ml of the antibody and histidine buffer, trehalose dihydrate, polysorbate 20, pH 5.5 was filled into the following syringes:

**Table 1:**

| **No.** | **Syringe size** | **Syringe barrel** | **Syringe type** | **Silicone level [mg]** | **Adhesive and tungsten** |
|---|---|---|---|---|---|
| **1** | 1.0 ml | Cyclo olefin polymer | Staked needle | no silicone | no |
| **2** | 1.0 ml | Borosilicate glass | Luer cones | Baked on silicone | no |
| **3** | 1.0 ml | Borosilicate glass | Luer cones | 0.6 | no |
| **4** | 0.5 ml | Cyclo olefin polymer | Luer cones | 0.6 | no |
| **5** | 0.5 ml | Borosilicate glass | Staked needle | 0.6 | yes |
| **6** | 0.5 ml | Borosilicate glass | Staked needle | 0.6 | yes |
| **7** | 1.0 ml | Polycarbonate | Luer cones | 1.0 | no |
| **8** | 1.0mL | Cyclo olefin polymer | Staked needle | no silicone | no |
| **9** | 1.0 ml | Borosilicate glass | Staked needle | <0.2 | yes |
| **10** | 1.0 ml | Borosilicate glass | Staked needle | 0.4-0.6 | yes |

The syringes from Table 1 were rotated from needle to stopper with a speed of 1 cycle/10 seconds at 40°C for five minutes, two weeks and four weeks or were subjected to five freeze/thaw cycles (+5 to -20°C with 1°C/min). Afterwards, the light obscuration was determined with the FlowCam PV bench top system (Fluid Imaging Technologies Inc., Maine, USA) using the System software (VisualSpreadsheet software, version 3.4.8) and the following parameters:

| | |
|---|---|
| Mode: | AutoImage |
| Priming Method: | machine prime |
| Flow Rate: | 0.040 ml/min |
| Recalibrations: | 0 |
| Stop Reason: | Sample Volume Processed |
| Sample Volume Aspirated: | 0.2900 ml |
| Sample Volume Processed: | 0.2893 ml |
| Fluid Volume Imaged: | 0.1822 ml |
| Efficiency: | 63.0% |
| Frame Rate: | 22.00 fps |
| Magnification: | 10X |
| Calibration Factor: | 0.6979 |
| Syringe Size: | 1.00 ml |

The results of the analysis are shown in Figures 1 and 2. The pre-filled silicone-free cycloolefin polymer syringes 1 and 8 have low particle levels under all conditions tested.

Additionally, the syringes listed in Table 2 were tested:

**Table 2:**

| **No.** | **Syringe size** | **Syringe barrel** | **Syringe type** | **Silicone level [mg]** | **Adhesive and tungsten** |
|---|---|---|---|---|---|
| **2** | 1.0 ml | Borosilicate glass | Luer cone | Baked-on | no |
| **11** | 1.0 ml | Cyclo olefin polymer | Luer cone | no silicone | no |
| **12** | 1.0 ml | Cyclo olefin polymer | Luer cone | 1.5 | no |
| **13** | 1.0 mL | Borosilicate glass | Staked needle | 0.7 | yes |
| **14** | 1.0 mL | Borosilicate glass | Staked needle | 0.25 ± 0.2 | yes |

The syringe No. 11 has the same barrel as the syringe 1 of Table 1, but is equipped with a Luer cone instead of a staked needle.

The syringes from Table 2 were incubated at 5°C for three, six and twelve months, at 25°C/60% relative humidity for two weeks, one month and three months and 40°C/75% relative humidity without rotation and then analyzed as described above for the syringes from Table 1.

The pre-filled silicone-free cycloolefin polymer syringe 11 has low particle levels under all conditions tested.

### 2. Determination of ranibizumab stability in different plastic and glass syringes subjected to stress conditions

The syringes as listed above in Table 1 were subjected to the stress conditions described in Example 1 for the syringes of Table 1. Further, the syringes as listed above in Table 2 were subjected to the conditions described in Example 1 for the syringes of Table 2.

Afterwards, the samples were analyzed by RP-HPLC for the presence of hydrophilic and hydrophobic species, by cation exchange chromatography for the presence of acidic and basic variants of the antibody and by size exclusion chromatography for the presence of aggregates.

### a) RP-HPLC analysis

The protein samples from the syringes were loaded onto a ZORBAX 300SB-C18, 4.6 x 100 mm, 3.5 µm column to detect hydrophilic and hydrophobic impurities.

The protein was eluted with a gradient of eluent A (0.1% trifluoroacetic acid in water) and eluent B (0.1% trifluoroacetic acid in 70% acetonitrile, 20% 1-propanol and 10% water) according to the following Table 3:

| Time [min] | Flow [mL/min] | Solvent composition Eluent A [%] | Solvent composition Eluent B [%] |
|---|---|---|---|
| 0 | 1.0 | 100 | 0 |
| 7 | 1.0 | 62.5 | 37.5 |
| 10 | 1.0 | 62.5 | 37.5 |
| 26 | 1.0 | 58.5 | 41.5 |
| 31 | 1.0 | 58.5 | 41.5 |
| 33 | 1.0 | 0 | 100 |
| 35 | 1.0 | 0 | 100 |
| 37 | 1.0 | 100 | 0 |
| 45 | 1.0 | 100 | 0 |

Eluted species were detected and displayed on a graph showing the concentration of the eluted species vs. time. The elution profile showed a main peak with the unmodified protein and some further peaks eluting before and after the main peak, representing hydrophilic and hydrophobic variants of the protein, respectively. The total area of all peaks as well as the area of the single peaks was determined. Figures 3 and 4 show the percentage of the peak area for hydrophilic species and hydrophobic species, respectively, in relation to the total peak area of the eluted species for the syringes of Table 1.

### b) Cation exchange analysis

The protein samples from the syringes were loaded onto a Dionex, BioLCProPac® WCX-10, 4.0 x 250 mm, 10 µm column to detect acidic and basic variants of the protein.

The protein was eluted with a gradient of mobile phase A (20 mM potassium phosphate buffer, ph 6.0) and mobile phase B (250 mM KCl, 20 mM potassium phosphate buffer, ph 6.0) according to the following Table 4:

| Time [min] | Solvent composition [%-B] | Solvent composition [mM KCl] |
|---|---|---|
| 0 | 0 | 0 |
| 3 | 0 | 0 |
| 33 | 50 | 125 |
| 35 | 50 | 125 |
| 36 | 0 | 0 |
| 40 | 0 | 0 |

Eluted species were detected and displayed on a graph showing the concentration of the eluted species vs. time. The elution profile showed a main peak with the unmodified protein and some further peaks eluting before and after the main peak, representing acidic and basic variants of the protein, respectively. The total area of all peaks as well as the area of the single peaks was determined. Figures 5 and 6 show the percentage of the peak area for acidic variants and basic variants, respectively, in relation to the total peak area of the eluted species for the syringes of Table 1.

### c) Size exclusion chromatography

The protein samples from the syringes were loaded onto a YMC-Pack Diol-200, 5 µm, 20 nm (8.0 x 300 mm) column to detect aggregates of the protein.

The protein was eluted by isocratic elution using 0.1 M potassium phosphate and 0.2 M sodium chloride. Eluted species were detected and displayed on a graph showing the concentration of the eluted species vs. time. The elution profile showed a main peak with the non-aggregated protein and some further peaks of the protein representing aggregated forms of the protein. The area of all peaks was determined. Figure 7 shows the percentage of peak area for the aggregates in relation to the total peak area of the eluted species for the syringes of Table 1.

From the results shown in Figures 3 to 7 it is apparent that the stability of ranibizumab is comparable in the pre-filled plastic syringes of the present invention (syringes 1 and 8) and the glass syringes under the conditions tested.

### 3. Determination of gliding forces in different plastic and glass Syringes containing ranibizumab

The syringes 1, 2, 7, 8 and 9 as listed above in Table 1 were tested for their stopper movement forces, i.e. the break loose force and the gliding force. To this end, 0.165 ml of a solution containing the anti-VEGF antibody ranibizumab in a concentration of 10 mg/ml as well as 10 mM histidine buffer, 10% (w/v) trehalose dihydrate, 0.01% (w/v) polysorbate 20, pH 5.5 were filled into the above syringes. Prior to testing, 30G x 0.5" needles were attached to the luer cone syringes. The testing was performed at a stopper speed of 190 mm/min over a travel length of 10.9 mm in a Tensile testing machine (TH2730, Thümler).

The results of the test are shown in Table 5 below.

**Table 5:**

| | | No. 1 | No. 2 | No. 7 | No. 8 | No. 10 |
|---|---|---|---|---|---|---|
| Break loose force | Average of 5 syringes | 3.4N | 3.4N | 0.4N | 3.5N | 3.5N |
| | Maximum individual value | 3.8N | 3.6N | 0.8N | 4.1N | 3.8N |
| Gliding force | Average of 5 syringes | 3.0N | 10.4N | 1.9N | 6.1N | 4.7N |
| | Maximum individual value | 4.0N | 11.2N | 2.3N | 6.5N | 5.IN |

The pre-filled silicone-free cycloolefin polymer syringes 1 and 8, i.e. the syringes of the present invention, have a gliding behavior which is comparable or even superior to that of syringes 2 and 10 which are coated with silicone oil.

Further, the syringes as listed above in Table 2 were tested for their stopper movement forces, i.e. the break loose force and the gliding force. To this end, 0.165 ml of a solution containing 10 mM histidine buffer, pH 5.5, 10% (w/v) trehalose dihydrate, 0.01% polysorbate 20 were filled into the above syringes. Prior to testing, 30G x 0.5" needles were attached to the luer cone syringes. The testing was performed at a stopper speed of 190 mm/min over a travel length of 10.9 mm in a Tensile testing machine (TH2730, Thümler).

The pre-filled silicone-free cycloolefin polymer syringe 11, i.e. a syringe of the present invention, has a gliding behavior which is comparable or even superior to that of syringes which are coated with silicone oil.

### 4. Determination of particles of different sizes in different plastic and glass syringes containing aflibercept and subjected to different conditions

400 µl of a solution of the VEGF receptor fusion protein aflibercept containing 1 mg/ml of the antibody and 10 mM sodium phosphate buffer, 40 mM sodium chloride, 5% (w/v) sucrose, 0.03% (w/v) polysorbate 20, pH 6.2 was filled into the following syringes:

**Table 6:**

| **No.** | **Syringe size** | **Syringe barrel** | **Syringe type** | **Silicone level [mg]** | **Adhesive and tungsten** |
|---|---|---|---|---|---|
| **2** | 1.0 ml | Borosilicate glass | Luer cone | Baked-on | no |
| **11** | 1.0 ml | Cyclo olefin polymer | Luer cone | no silicone | no |
| **12** | 1.0 ml | Cyclo olefin polymer | Luer cone | 1.5 | no |
| **13** | 1.0 mL | Borosilicate glass | Staked needle | 0.7 | yes |
| **14** | 1.0 mL | Borosilicate glass | Staked needle | 0.25 ± 0.2 | yes |

The syringes from Table 6 were rotated from needle to stopper with a speed of 1 cycle/10 seconds at 40°C for five minutes, two weeks and four weeks or were subjected to five freeze/thaw cycles (+5 to -20°C with 1°C/min). The syringes were also incubated at 5°C for three, six and twelve months, at 25°C/60% relative humidity for two weeks, one month and three months and 40°C/75% relative humidity without rotation and then analyzed as described above for the syringes from Table 1.

### 5. Determination of gliding forces in different plastic and glass syringes containing aflibercept

The syringes as listed above in Table 6 were tested for their stopper movement forces, i.e. the break loose force and the gliding force. To this end, 0.165 ml of a solution containing the VEGF receptor fusion protein aflibercept in a concentration of 40 mg/ml and 10 mM sodium phosphate buffer, 40 mM sodium chloride, 5% (w/v) sucrose, 0.03% (w/v) polysorbate 20, pH 6.2 was filled into the above syringes. Prior to testing, 30G x 0.5" needles were attached to the luer cone syringes. The testing was performed at a stopper speed of 190 mm/min over a travel length of 10.9 mm in a Tensile testing machine (TH2730, Thümler).

Further embodiments of the invention are described herein:
1. Pre-filled syringe containing a liquid formulation of a VEGF antagonist and comprising a syringe barrel, wherein the syringe barrel is made of plastic and is silicone-free.
2. Pre-filled syringe according to item 1, wherein the VEGF antagonist is an anti-VEGF antibody or an antigen-binding fragment of such antibody or a VEGF receptor fusion protein.
3. Pre-filled syringe according to item 2, wherein the anti-VEGF antibody is ranibizumab or aflibercept.
4. Pre-filled syringe according to any of the preceding items, wherein the antagonist concentration is 1 to 100 mg/ml.
5. Pre-filled syringe according to any of the preceding items, containing less than 50 particles per ml of the liquid formulation having a diameter of 10 µm or greater.
6. Pre-filled syringe according to any of the preceding items, containing less than 5 particles per ml of the liquid formulation having a diameter of 25 µm or greater.
7. Pre-filled syringe according to any of the preceding items, having a slide force of less than or equal to 10N.
8. Pre-filled syringe according to any of the preceding items, further comprising a silicone-free stopper.
9. Pre-filled syringe according to any of the preceding items, wherein the syringe barrel is made of cycloolefin polymer or cycloolefin copolymer.
10. Pre-filled syringe according to any of the preceding items, wherein the syringe barrel comprises an internal coating other than a silicone coating.
11. Pre-filled syringe according to any of the preceding items, comprising a staked needle.
12. Kit comprising one or more pre-filled syringes of any of the preceding items.
13. Pre-filled syringe of any of items 1 to 11 for use in administering a liquid formulation of a VEGF antagonist to a patient having an ocular disease.
14. Pre-filled syringe for the use according to item 13, wherein the ocular disease is selected from the group consisting of age-related macular degeneration (AMD), visual impairment due to diabetic macular oedema (DME), visual impairment due to macular oedema secondary to retinal vein occlusion (branch RVO or central RVO), diabetic retinopathy in patients with diabetic macular edema or visual impairment due to choroidal neovascularisation (CNV) secondary to pathologic myopia.
15. Pre-filled syringe for the use according to item 13 or 14, wherein a volume of 30 to 100 µl of the liquid formulation is administered to the patient.

## Claims

1. Pre-filled syringe containing a liquid formulation of a VEGF antagonist and comprising a syringe barrel for use in the intravitreal injection of said VEGF antagonist, wherein the syringe barrel is made of plastic and is silicone-free and wherein the VEGF antagonist is an anti-VEGF antibody or an antigen-binding fragment of such antibody or a VEGF receptor fusion protein, wherein the anti-VEGF antibody or VEGF receptor fusion protein retains its biological activity when stored at a temperature of 2 to 8°C for at least six months.

2. Pre-filled syringe according to claim 1, wherein the anti-VEGF antagonist is ranibizumab or aflibercept.

3. Pre-filled syringe according to any of the preceding claims, wherein the antagonist concentration is 1 to 100 mg/ml.

4. Pre-filled syringe according to any of the preceding claims, containing less than 50 particles per ml of the liquid formulation having a diameter of 10 µm or greater.

5. Pre-filled syringe according to any of the preceding claims, containing less than 5 particles per ml of the liquid formulation having a diameter of 25 µm or greater.

6. Pre-filled syringe according to any of the preceding claims, having a slide force of less than or equal to 10N.

7. Pre-filled syringe according to any of the preceding claims, further comprising a silicone-free stopper.

8. Pre-filled syringe according to any of the preceding claims, wherein the syringe barrel is made of cycloolefin polymer or cycloolefin copolymer.

9. Pre-filled syringe according to any of the preceding claims, wherein the syringe barrel comprises an internal coating other than a silicone coating.

10. Pre-filled syringe according to any of the preceding claims, comprising a staked needle.

11. Kit comprising one or more pre-filled syringes of any of the preceding claims.

12. Pre-filled syringe of any of claims 1 to 10 for use in administering a liquid formulation of a VEGF antagonist to a patient having an ocular disease.

13. Pre-filled syringe for the use according to claim 12, wherein the ocular disease is selected from the group consisting of age-related macular degeneration (AMD), visual impairment due to diabetic macular oedema (DME), visual impairment due to macular oedema secondary to retinal vein occlusion (branch RVO or central RVO), diabetic retinopathy in patients with diabetic macular edema or visual impairment due to choroidal neovascularisation (CNV) secondary to pathologic myopia.

14. Pre-filled syringe for the use according to claim 12 or 13, wherein a volume of 30 to 100 µl of the liquid formulation is administered to the patient.
